(19) Europäisches Patentamt
European Patent Office
Office européen des brevets

(11) Numéro de publication : **0 537 242 B1**

# (12) FASCICULE DE BREVET EUROPEEN

(45) Date de publication du fascicule du brevet : **13.04.94 Bulletin 94/15**

(51) Int. Cl.$^5$ : **C07F 13/00,** C07B 59/00, A61K 49/02

(21) Numéro de dépôt : **91912410.7**

(22) Date de dépôt : **03.07.91**

(86) Numéro de dépôt international : **PCT/FR91/00536**

(87) Numéro de publication internationale : **WO 92/00982 23.01.92 Gazette 92/03**

## (54) PROCEDE DE PREPARATION DE COMPLEXES NITRURO DE METAUX DE TRANSITION.

(30) Priorité : **04.07.90 FR 9008473**

(43) Date de publication de la demande : **21.04.93 Bulletin 93/16**

(45) Mention de la délivrance du brevet : **13.04.94 Bulletin 94/15**

(84) Etats contractants désignés : **AT BE CH DE DK ES FR GB GR IT LI LU NL SE**

(56) Documents cités :
**WO-A-89/08657**
**INTERNATL. JOURNAL OF RADIATION / Applications & Instrumentation Part A, vol. 39, no. 3, 1988, Pergamon Journals Ltd., Marsh Barton, Exeter (GB); C.M. KENNEDY et al., pp. 213-225**

(73) Titulaire : **CIS BIO INTERNATIONAL**
**RN 306**
**F-91000 Saclay (FR)**

(72) Inventeur : **PASQUALINI, Roberto**
**223, avenue Victor-Hugo**
**F-92140 Clamart (FR)**
Inventeur : **COMAZZI, Véronique**
**15, rue Maurice-Hartmann**
**F-92130 Issy-les-Moulineaux (FR)**
Inventeur : **BELLANDE, Emmanuel**
**7, rue du Trou Mahet**
**F-91160 Champlan (FR)**

(74) Mandataire : **Des Termes, Monique et al**
**Société Brevatome 25, rue de Ponthieu**
**F-75008 Paris (FR)**

EP 0 537 242 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un procédé de préparation de complexes de métaux de transition, utilisables en particulier comme produits radiopharmaceutiques ou pour la synthèse de nouveaux produits radiopharmaceutiques.

De façon plus précise, elle concerne la préparation de complexes nitruro de métaux de transition comportant une partie M≡N, dans laquelle M représente un métal de transition.

On précise que l'on entend par métal de transition un métal dont la couche d est partiellement remplie dans le degré d'oxydation usuel de ce métal. Il s'agit des éléments remplissant les périodes III à XII du tableau périodique des éléments à 18 colonnes.

A titre d'exemple de tels métaux, on peut citer Tc, Ru, Co, Pt, Fe, Os, Ir, W, Re, Cr, Mo, Mn, Ni, Rh, Pd, Nb et Ta.

L'invention concerne en particulier la préparation de complexes nitruro de métaux de transition radioactifs, utilisables comme produits radiopharmaceutiques pour le diagnostic ou la thérapie.

Parmi les complexes utilisables pour le diagnostic, on peut citer en particulier les complexes de technétium 99m. Les complexes utilisables pour la thérapie peuvent être par exemple des complexes de rhénium.

Les produits radiopharmaceutiques utilisant le radionucléide $^{99m}Tc$ sont des composés fréquemment utilisés en médecine nucléaire pour le diagnostic en raison des caractéristiques physiques et chimiques de ce radionucléide.

En effet, celui-ci donne uniquement une émission gamma, présente une énergie gamma optimale pour la détection externe (140 kev) et une courte demi-vie physique (6,02h), ce qui permet de ne délivrer qu'une faible dose d'irradiation au patient. Par ailleurs, le coût de ce radioélément est peu élevé et il est disponible commercialement. Enfin, la richesse de la chimie du technétium permet d'obtenir une grande variété de produits radiopharmaceutiques.

En effet, comme il est indiqué par E. DEUTSCH et al. dans : Progr. Inorg. Chem. (Australia), vol. 30, PP 76-106, 1983, le technétium peut former des complexes très variés avec de nombreux ligands, à des états d'oxydation allant de VII à -I et des nombres de coordination allant de 4 à 9.

Parmi les nombreux complexes de ce document, on trouve des complexes de $^{99m}Tc$ et $^{99}Tc$ à l'état d'oxydation V comportant une partie Tc≡N.

Des complexes de ce type peuvent être préparés par le procédé décrit par J. Baldas et Col. dans J. Chem. Soc. Dalton Trans. 1981, pp. 1798-1801 ; dans Int. J. Appl. Radiat. Isot. 36 (1985), pp. 133-139 et dans la demande de brevet internationale W085/03063. Ce procédé consiste à préparer tout d'abord un composé de formule $R^+$ $[^{99m}Tc{\equiv}NX_4]$ dans laquelle $R^+$ est un cation tel que le sodium ou l'ammonium et X représente un atome d'halogène tel que Cl ou Br, et à faire réagir ensuite ce composé avec un ligand approprié pour obtenir le complexe de $^{99m}Tc$ utilisable comme produit radiopharmaceutique.

Le complexe $R^+[^{99m}Tc{\equiv}NX_4]^-$ est intéressant car il est très stable à l'hydrolyse et peut être utilisé sans modification de la partie Tc≡N pour des réactions de substitution avec d'autres ligands, ce qui permet d'obtenir une grande diversité de complexes de technétium.

Le procédé connu actuellement pour préparer le composé intermédiaire $R^+[^{99m}TCNX_4]^-$ consiste à faire réagir un pertechnétate tel que le pertechnétate de sodium avec de l'azoture de sodium et un hydracide halogéné tel que l'acide chlorhydrique.

Pour effectuer cette réaction, on évapore à sec une solution de pertechnétate de sodium ($^{99m}Tc$)en utilisant un évaporateur rotatif, puis on ajoute au résidu sec, de l'azoture de sodium et de l'acide chlorhydrique concentré. On porte au reflux pendant environ 5min pour compléter la réduction et détruire l'excès d'azoture et on évapore de nouveau à sec en utilisant un évaporateur rotatif. On obtient ainsi un résidu contenant le composé $R^+(^{99m}Tc{\equiv}NCl_4)^-$.

Ce procédé est difficilement applicable à la fabrication de trousses à usage médical car il est long et comprend au moins trois étapes dans lesquelles on utilise deux fois un évaporateur rotatif, ce qui n'est pas facile à mettre en oeuvre dans un service hospitalier de médecine nucléaire. De plus, ce procédé est difficilement utilisable pour la fabrication de trousses à usage médical car la stérilité et l'apyrogénéité des solutions sont difficilement contrôlables tout au long des opérations. De ce fait, le produit finalement obtenu doit être stérilisé après marquage par le $^{99m}Tc$, soit par passage sur une membrane stérilisante, soit par stérilisation à la chaleur, et il doit subir un contrôle d'absence de pyrogène avant l'injection sur l'homme.

Dans le cas de la préparation de complexes utilisables pour la thérapie, il est important d'utiliser un procédé de préparation conduisant à un rendement élevé en produits désirés.

Or, dans le cas du produit mis au point par Baldas ou Griffith (Coord. Chem. Rev. vol 8, 1972, pp. 369-396) pour la préparation de complexes nitruro de technétium, on ne peut obtenir des rendements élevés en complexes.

Il en est de même avec les procédés généralement utilisés pour préparer des produits radiopharmaceutiques à base de rhénium.

Dans la demande internationale WO 89/08657, on a décrit un procédé de préparation de complexes nitruro d'un métal de transition qui pallie les inconvénients du procédé décrit par Baldas. Ce procédé consiste à faire réagir un composé oxygéné d'un métal de transition M avec un premier ligand choisi dans le groupe des phosphines et polyphosphines aliphatiques et aromatiques, substituées ou non substituées, et un second ligand azoté constitué soit par un azoture d'ammonium ou de métal pharmaceutiquement acceptable, soit par un composé azoté comportant un motif ⋝N-N⋜ dans lequel les N sont reliés à des atomes d'hydrogène et/ou à des groupements organiques monovalents par l'intermédiaire d'un atome de carbone ou dans lequel l'un des N est relié à l'atome de carbone d'un groupement organique bivalent par l'intermédiaire d'une double liaison et l'autre N est relié à des atomes d'hydrogène et/ou à des groupements organiques monovalents par l'intermédiaire d'un atome de carbone.

Selon ce procédé, on peut obtenir facilement un complexe nitruro d'un métal de transition car il suffit de mélanger les réactifs décrits ci-dessus pour former le complexe nitruro. On peut de plus former d'autres produits radiopharmaceutiques à partir de ce complexe nitruro par réaction d'échange avec un troisième ligand.

Bien que ce procédé soit tout à fait satisfaisant et permette de réaliser de nombreux complexes nitruro de technétium, les recherches ont été poursuivies pour tenter de simplifier encore le mode de préparation des complexes nitruro de technétium et utiliser plutôt des réactifs dont les effets pharmacologiques et biologiques soient bien connus afin de limiter les études préliminaires nécessaires en vue de la commercialisation de nouveaux produits radiopharmaceutiques.

La présente invention a précisément pour objet un procédé de préparation de complexes nitruro de métaux de transition, basé sur l'emploi d'agents réducteurs bien connus qui ont été souvent utilisés pour la préparation d'autres complexes de technétium que les complexes nitruro.

Selon l'invention, le procédé de préparation d'un produit comprenant un complexe nitruro d'un métal de transition comportant une partie M≡N ,avec M représentant un métal de transition, consiste à faire réagir en solution un composé oxygéné du métal de transition M avec :

1°) un premier ligand azoté constitué soit par un azoture d'ammonium ou de métal pharmaceutiquement acceptable, soit par un composé azoté comportant un motif ⋝N-N⋜ dans lequel les N sont reliés à des atomes d'hydrogène et/ou à des groupements organiques monovalents, par exemple par l'intermédiaire d'un atome de carbone ou d'un atome de S, ou dans lequel l'un des N est relié à l'atome de carbone d'un groupement organique bivalent par l'intermédiaire d'une double liaison et l'autre N est relié à des atomes d'hydrogène et/ou a des groupements organiques monovalents, par exemple par l'intermédiaire d'un atome de carbone, et

2°) un agent réducteur constitué, soit par du dithionite d'ammonium ou d'un métal pharmaceutiquement acceptable, soit par de l'étain (II) présent sous forme ionique dans la solution.

Dans ce procédé, on utilise donc à la place des phosphines ou polyphosphines de la demande internationale WO 89/08657, soit de l'étain (II), soit un dithionite d'ammonium ou d'un métal pharmaceutiquement acceptable.

Ceci simplifie considérablement la mise au point de nouveaux produits radiopharmaceutiques tels que les produits à base de technétium, car la toxicité et les effets biologiques de l'étain, qui peut être utilisé sous forme de sel d'étain comme le chlorure d'étain, sont bien connus dans ce domaine, ce qui n'est pas du tout le cas pour les phosphines et polyphosphines de la demande internationale WO 89/08657.

En effet, l'étain est utilisé depuis longtemps pour la préparation de complexes de technétium du type pyrophosphate, méthylènediphosphonate et hydroxyméthylènediphosphonate, comme il est décrit dans International Journ. of Radiation/Application, and Instrumentation Part A, vol. 39, N° 3, pp. 213-225

De plus, les composés d'étain (11) et les dithionites sont généralement solubles dans l'eau, ce qui simplifie la préparation du complexe nitruro de métal de transition puisque l'on peut opérer en solution aqueuse, c'est-à-dire dans un milieu approprié pour l'administration à l'homme et aux êtres vivants.

Par ailleurs, ces composés sont stables et ne réagissent pas avec des substrats tels que les fragments $F(ab')_2$ des anticorps.

Aussi, selon une variante de réalisation du procédé de l'invention, on utilise de plus un deuxième ligand organique, un anticorps, un fragment d'anticorps, une protéine ou un peptide qui permet de conférer au produit final les propriétés biologiques recherchées, en particulier le tropisme voulu.

Dans ce cas, on fait réagir en solution un composé oxygéné du métal de transition M avec :

1°) un premier ligand azoté constitué, soit par un azoture d'ammonium ou de métal pharmaceutiquement acceptable, soit par un composé azoté comportant un motif ⋝N-N⋜ dans lequel les N sont reliés à des atomes d'hydrogène et/ou à des groupements organiques monovalents, par exemple par l'intermédiaire d'un atome de carbone ou d'un atome de S, ou dans lequel l'un des N est relié à l'atome de carbone d'un groupement organique bivalent par l'intermédiaire d'une double liaison et l'autre N est relié à des atomes d'hydrogène et/ou à des groupements organiques monovalents, par exemple par l'intermédiaire d'un atome de carbone ;

2°) un agent réducteur constitué soit par un dithionite d'ammonium ou de métal pharmaceutiquement acceptable, soit par de l'étain II présent sous forme ionique dans la solution ; et

3°) un deuxième ligand organique à groupement nucléophile, un anticorps monoclonal, un fragment d'anticorps, une protéine ou un peptide.

Lorsqu'on utilise ce deuxième ligand organique, cet anticorps, cette protéine ou ce peptide, il se produit probablement une réaction d'échange entre le premier ligand azoté qui a contribué à la préparation de la partie M≡N et le deuxième ligand organique, l'anticorps, la protéine ou le peptide.

Ces deux réactions peuvent être effectuées simultanément en faisant réagir dans la solution tous les réactifs en même temps. Toutefois, on opère généralement de préférence en deux étapes. Ainsi, dans une première étape on fait réagir le composé oxygéné du métal de transition avec le premier ligand et l'agent réducteur, et dans une deuxième étape, on fait réagir le produit obtenu à la suite de la première étape avec le troisième ligand, l'anticorps monoclonal, le fragment d'anticorps, la protéine ou le peptide.

Le procédé de l'invention peut être mis en oeuvre de différentes façons qui dépendent en particulier de l'agent réducteur utilisé ainsi que du choix des premier et deuxième ligands.

Selon un premier mode de réalisation de l'invention, on utilise comme agent réducteur l'étain (II) et on l'introduit dans la solution à partir d'un ou plusieurs réactifs capables de le maintenir sous forme ionique en présence du premier et éventuellement du second ligands pour éviter la précipitation des complexes d'étain susceptibles de se former avec le premier ou le second ligand.

Ainsi, on peut introduire l'étain sous la forme de sel d'étain (II) lorsque l'anion de ce sel d'étain a un meilleur pouvoir complexant sur l'étain que les autres réactifs présents dans la solution, c'est-à-dire le premier ligand azoté et éventuellement le second ligand.

A titre d'exemple, le sel d'étain peut être le tartrate ou l'oxalate d'étain.

On peut aussi introduire l'étain (II) et le maintenir sous forme ionique dans la solution à partir d'autres sels d'étain tels que les halogénures et le sulfate d'étain, en particulier le chlorure d'étain, à condition d'ajouter simultanément à la solution un agent complexant ayant un pouvoir complexant vis-à-vis de l'étain plus fort que ceux du ligand azoté et du second ligand éventuellement utilisé.

Dans ce cas, on ajoute du chlorure d'étain (II) et un agent complexant approprié à la solution du composé oxygéné de métal de transition et du premier ligand azoté.

A titre d'exemple d'agents complexants utilisables, on peut citer les pyrophosphates de métal alcalin ou d'ammonium, les glucoheptonates de métal alcalin ou d'ammonium, les diéthylène triamino pentacétates de métal alcalin ou d'ammonium, les éthylènediaminotétracétates de métal alcalin ou d'ammonium, les diamino-1,2 propane N,N,N',N'-tétracétates de métal alcalin ou d'ammonium, les gluconates de métal alcalin ou d'ammonium, les méthylènediphosphonates de métal alcalin ou d'ammonium, les hydroxyméthylènediphosphonates de métal alcalin ou d'ammonium et les citrates de métal alcalin ou d'ammonium.

D'une façon générale, on peut utiliser comme agent complexant tous les complexants de l'étain tels que les phosphonates, les polyphosphates et les acides polyaminocarboxyliques.

Selon un second mode de réalisation du procédé de l'invention, l'agent réducteur est un dithionite d'ammonium ou d'un métal pharmaceutiquement acceptable.

Les dithionites de métaux pharmaceutiquement acceptables peuvent être en particulier les dithionites de métaux alcalins, par exemple le dithionite de sodium.

Comme on l'a vu précédemment, on réalise de préférence, selon l'invention, la réaction entre le composé oxygéné du métal de transition, le premier ligand azoté et l'agent réducteur dans une solution aqueuse dont le pH est ajusté à une valeur appropriée. On peut toutefois aussi opérer dans des solutions alcooliques ou hydroalcooliques.

On peut opérer dans une large gamme de pH, allant par exemple de 1,5 à 11 ; toutefois, on opère de préférence à un pH de 7 à 8.

Pour réaliser la réaction, on peut introduire aseptiquement une solution stérile du composé oxygéné de métal de transition et lui ajouter une solution stérile du ligand azoté et de l'agent réducteur dont le pH a été ajusté à la valeur voulue par addition d'acide, de base ou d'un tampon approprié. On peut ensuite effectuer la réaction à la température ambiante ou à une température supérieure, allant par exemple de 50 à 100°C, pendant des durées variables qui dépendent en particulier de la température utilisée.

Généralement, on opère avec des rapports molaires composé oxygéné du métal de transition / premier ligand azoté de $10^{-9}$ à $10^{-2}$.

Les quantités de 1er ligand azoté et d'agent réducteur utilisées peuvent être très faibles et varier dans une large gamme allant par exemple de 0,03 à 30µmol/ml pour le 1er ligand azoté et de 0,02 à 2µmol/ml pour l'agent

réducteur.

Après cette réaction, on peut ajouter le deuxième ligand, l'anticorps monoclonal, le fragment d'anticorps, la protéine ou le peptide, et le laisser réagir à la température ambiante ou à une température supérieure pouvant aller par exemple de 37 à 45°C pour des anticorps ou de 100°C pour des molécules résistant à cette température, pendant des durées variables qui dépendent en particulier de la température utilisée.

Généralement, on opère avec des rapports molaires composé oxygéné du métal de transition / second ligand de $10^{-9}$ à $10^{-2}$. Dans le cas où l'on utilise un anticorps, le rapport molaire composé oxygéné du métal de transition / anticorps est de $10^{-3}$ à $10^{-1}$.

Dans cette deuxième étape, on peut aussi ajuster le pH de la solution à une valeur appropriée en introduisant le second ligand dans une solution aqueuse ayant un pH adapté. Le pH utilisé peut varier dans une large gamme, par exemple de 3,5 à 11. De préférence, on opère au pH physiologique.

On peut aussi réaliser cette deuxième étape, en présence d'autres additifs, par exemple d'un agent complexant permettant d'éviter la réaction de l'étain avec le deuxième ligand utilisé, en particulier pour éviter la formation de précipités.

Comme précédemment, on réalisé de préférence cette deuxième étape en solution aqueuse, mais on pourrait également la réaliser en solution alcoolique ou hydroalcoolique, ou encore effectuer la première étape et la deuxième étape dans des solutions différentes, par exemple la première étape en solution aqueuse et la deuxième étape en solution alcoolique ou hydroalcoolique ou l'inverse.

Pour réaliser la deuxième étape, on peut utiliser divers ligands organiques car leur choix dépend uniquement des propriétés que l'on veut conférer au complexe final.

Ainsi, on peut utiliser comme second ligand, des amines, des thiols, des thioéthers, des oximes, des phosphines et des ligands polyfonctionnels du type polyaminopolythiols.

A titre d'exemple, on peut utiliser en particulier les composés répondant à la formule :

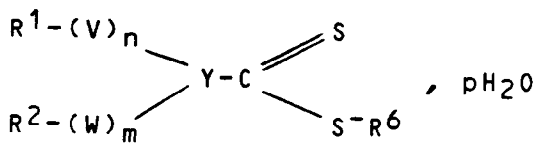 (X)

dans laquelle V et W qui peuvent être identiques ou différents, représentent 0, S ou Se, n et m qui peuvent être identiques ou différents, sont égaux à 0 ou à 1, Y représente N, P ou As, et $R^1$ et $R^2$ qui peuvent être identiques ou différents, représentent un radical alkyle linéaire ou ramifié de 1 à 10 atomes de carbone, non substitué ou substitué par des groupements $-O-R^3$, $OOC-R^3$, $OCNR^4R^5$ ou $-NR^4R^5$ dans lesquels $R^3$ est un radical alkyle linéaire ou ramifié de 1 à 5 atomes de carbone et $R^4$ et $R^5$ qui peuvent être identiques ou différents, sont des atomes d'hydrogène ou des radicaux alkyle linéaires ou ramifiés de 1 à 5 atomes de carbone, ou dans laquelle $R^1$ et $R^2$ forment ensemble un cycle hydrocarboné contenant éventuellement un ou plusieurs hétéroatomes, et $R^6$ est un ion de métal alcalin, $H^+$ ou $NH_4^+$, et p=0 ou est un nombre entier allant de 1 à 5.

Des ligands de ce type sont décrits en particulier dans la demande internationale WO/90 06137 pour la réalisation de produits radiopharmaceutiques ayant notamment un tropisme cardiaque.

A titre d'exemple de tels ligands, on peut citer le diéthyldithiocarbamate de sodium.

A titre d'exemple d'autres deuxièmes ligands utilisables dans le procédé de l'invention, on peut citer la thioquinoléine, la pénicillamine, l'éthyl dithiocarboxylate de sodium, l'isopropylxanthate de sodium et le diéthyl dithiophosphinate de sodium.

Dans cette deuxième étape, on peut aussi utiliser un anticorps monoclonal ou un fragment d'anticorps.

Lorsque la réaction est effectuée avec un anticorps monoclonal ou un fragment d'anticorps, on peut préparer de cette façon un anticorps marqué par un métal de transition. Pour cette réaction, l'anticorps monoclonal ou le fragment d'anticorps utilisé peut être activé, par exemple par prétraitement avec l'amino éthane thiol ou le dithiothréitol, pour convertir les liaisons disulfure en groupe sulfhydryle.

On peut utiliser de nombreux types d'anticorps, en particulier ceux capables de se lier à la partie M≡N par des atomes de soufre. A titre d'exemples de tels anticorps, on peut citer les anticorps anti-ACE (anti-antigène carcinoembryonnaire), les anticorps anti-carcinome ovarien (OC125), les anticorps anticolorectal, anti-fibrine, anti-myosine.

L'anticorps ou le fragment d'anticorps marqué obtenu est très intéressant, par exemple pour la détection des tumeurs. En effet, après réaction avec le complexe de métal de transition, l'anticorps monoclonal ou le fragment d'anticorps est lié à l'élément de transition tel que le technétium 99m, mais il peut réagir avec les antigènes correspondants. Ainsi, la spécificité de l'anticorps est maintenue et l'anticorps marqué est stable. Aussi, on peut utiliser cet anticorps marqué pour la détection de tumeurs car il se dirigera naturellement vers l'antigène

correspondant et permettra la visualisation des tumeurs.

Dans la deuxième étape, on peut aussi utiliser une protéine ou un peptide en vue de conférer au produit radiopharmaceutique obtenu le tropisme voulu .

Selon l'invention, le premier ligand azoté utilisé qui permet la formation du complexe nitruro de métal de transition peut être de différents types. Ainsi, on peut utiliser soit un azoture d'ammonium ou de métal alcalin, soit un composé azoté. Les composés azotés peuvent répondre à la formule :

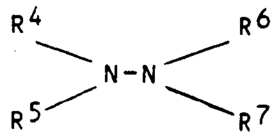

dans laquelle $R^4$, $R^5$, $R^6$ et $R^7$ qui peuvent être identiques ou différents, représentent un atome d'hydrogène ; un radical alkyle ; un radical aryle ; un radical alcoxy ; un radical alkyle substitué par au moins un groupement choisi parmi les radicaux hydroxy, carboxy, amino, amido et mercapto ; un radical aryle substitué par au moins un groupement choisi parmi les atomes d'halogène et les radicaux alcoxy, hydroxy, amino, mercapto et amino substitué par au moins un radical alkyle ; un radical répondant aux formules :

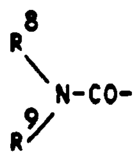 , 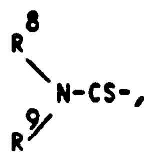 , ou 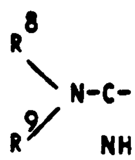

dans lesquelles $R^8$ et $R^9$ qui peuvent être identiques ou différents, représentent un atome d'hydrogène, un radical alkyle, ou un radical amino ; un radical de formule :

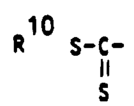

dans laquelle $R^{10}$ représente un atome d'hydrogène, un radical alkyle ou un radical aryle ; un radical de formule $R^{11}$-CO- avec $R^{11}$ représentant un radical alkyle, un radical alcoxy, un radical aryle non substitué ou substitué par au moins un groupement choisi parmi les atomes d'halogène et les radicaux hydroxy, ou un radical dérivé d'un hétérocycle non substitué ou substitué par au moins un groupement choisi parmi les atomes d'halogène et les radicaux hydroxy, ou un radical dérivé d'un hétérocycle non substitué ou substitué par au moins un groupement choisi parmi les atomes d'halogène et les radicaux hydroxy ;ou dans laquelle $R^4$ et $R^5$ peuvent former ensemble un radical bivalent de formule :

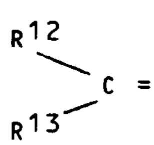

dans laquelle $R^{12}$ représente $-CH_2-NH_2-$, un radical aryle non substitué ou substitué par au moins un groupement choisi parmi les atomes d'halogène et les radicaux hydroxy, alcoxy, amino, mercapto et amino substitué par au moins un radical alkyle, ou un radical dérivé d'un hétérocycle non substitué ou substitué par un ou plusieurs groupements choisis parmi les atomes d'halogène et les radicaux hydroxy, alcoxy, amino, mercapto et amino substitué par au moins un radical alkyle et, $R^{13}$ représente un atome d'hydrogène, un radical alkyle ou un radical alkyle substitué par au moins un groupement choisi parmi les radicaux hydroxy, carboxy, amino, amido et mercapto ; et $R^6$ et $R^7$ ont la signification donnée ci-dessus.

Des exemples de tels composés azotés sont décrits en détail dans la demande internationale WO 89/08657.

On peut aussi utiliser dans l'invention, comme premier ligand azoté, des ligands répondant à la formule :

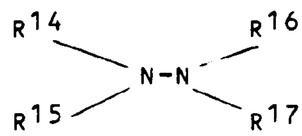

dans laquelle $R^{14}$, $R^{15}$ et $R^{16}$ qui peuvent être identiques ou différents, représentent un atome d'hydrogène, un radical alkyle, un radical aryle, un radical alcoxy, un radical alkyle substitué par au moins un groupement choisi parmi les radicaux hydroxy, carboxy, amino, amido et mercapto ou un radical aryle substitué par au moins un groupement choisi parmi les atomes d'halogène et les radicaux alcoxy, hydroxy, amino, mercapto et amino substitué par au moins un radical alkyle,

$R^{17}$ représente un radical répondant aux formules

$$-SO_2R^{18} \text{ ou } -CO\,R^{19}$$

dans lesquelles $R^{18}$ est un groupe phényle non substitué ou substitué par au moins un substituant choisi parmi les atomes d'halogène et les radicaux alkyle, et

$R^{19}$ est un atome d'hydrogène, $-NH_2$ ou un radical choisi parmi les radicaux alkyle, les radicaux alkyle substitués par au moins un groupement choisi parmi les groupements cyano, pyridyle et $-CO-NH-NH_2$, le radical phényle, le radical phényle substitué par au moins un substituant choisi parmi -OH, $NH_2$ et les radicaux alkyle et alcoxy.

Dans cette formule, les radicaux alkyle et alcoxy peuvent être des radicaux linéaires ou ramifiés, de préférence de 1 à 4 atomes de carbone, par exemple le radical méthyle ou méthoxy.

Les radicaux aryle sont des radicaux dérivés d'un noyau par élimination d'un atome d'hydrogène, par exemple les radicaux phényle et naphtyle.

L'utilisation de tels ligands est intéressante car elle permet de réaliser la première réaction à la température ambiante pour former le produit intermédiaire, et de préparer ensuite un produit radiopharmaceutique d'une pureté radiochimique d'au moins 95 %, par réaction de ce produit intermédiaire avec un deuxième ligand.

De préférence, lorsqu'on veut réaliser la réaction à la température ambiante, le premier ligand azoté répond à la formule précitée :

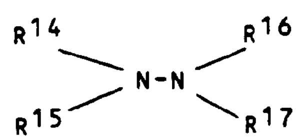

dans laquelle $R^{14}$, $R^{15}$ et $R^{16}$ sont des atomes d'hydrogène, $R^{17}$ représente le radical de formule $SO_2R^{18}$ avec $R^{18}$ ayant la signification donnée ci-dessus, ou le radical de formule -COH, $-COCH_2CH_2CONHNH_2$ ou

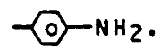

Lorsque $R^{17}$ représente $SO_2R^{18}$, $R^{18}$ peut être le radical phényle, le radical p-méthylphényle, le radical trichloro-1,3,5 phényle ou le radical triméthyl-1,3,5 phényle.

Les ligands azotés répondant aux formules précitées peuvent également être utilisés à la température ambiante en procurant les mêmes avantages dans le procédé décrit dans WO 89/08657. Dans ce cas, on forme le produit intermédiaire par réaction d'un composé oxygéné du métal de transition avec un ligand choisi dans le groupe des phosphines et polyphosphines, aliphatiques et aromatiques, substituées ou non substituées, et le ligand azoté de formule

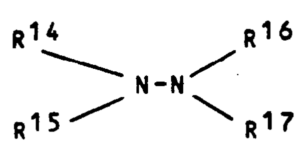

décrit ci-dessus.

Selon l'invention, le composé oxygéné de métal de transition est avantageusement un pertechnétate-99m de métal alcalin ou d'ammonium, ou un perrhénate-186 ou -188 de métal alcalin ou d'ammonium.

L'invention a également pour objet une trousse pour la préparation d'un produit radiopharmaceutique comprenant un complexe nitruro de métal de transition. Cette trousse comprend :

- un premier flacon contenant le premier ligand azoté et l'agent réducteur, et un second flacon contenant un deuxième ligand organique à groupement nucléophile, un anticorps monoclonal, un fragment d'anticorps, une protéine ou un peptide.

Ainsi, avec cette trousse, on peut préparer directement le produit radiopharmaceutique voulu, dans un service hospitalier de médecine nucléaire, en mélangeant le contenu des deux flacons après avoir ajouté par exemple au premier flacon une solution de pertechnétate de métal alcalin ou d'ammonium.

Le premier ligand, l'agent réducteur et le second ligand, peuvent être présents respectivement dans les flacons sous forme liquide ou sous forme lyophilisée.

D'autres caractéristiques et avantages de l'invention apparaitront mieux à la lecture des exemples suivants donnés bien entendu à titre illustratif et non limitatif.

<u>Exemple 1</u> : <u>Préparation d'un complexe nitruro de $^{99m}$Tc.</u>

Dans cet exemple on utilise comme agent réducteur de l'étain (II) introduit à partir de chlorure d'étain et d'un agent complexant constitué par le pyrophosphate de sodium, afin de maintenir l'étain sous forme ionique dans la solution. La réaction est effectuée en solution aqueuse et le pH est ajusté à une valeur de 7 par addition d'un tampon phosphate.

Dans un flacon du type pénicilline, on introduit :

- 0,5 à 3ml d'une solution stérile de pertechnétate de sodium (technétium-99m) correspondant à une radioactivité allant de 18MBq à 18,5GBq (0,5 à 500mCi),
- 1ml d'un tampon phosphate ayant une concentration molaire allant de 0,1 à 0,5M et un pH de 7,4 à 8,
- 0,1 à 0,5ml d'une solution aqueuse contenant :
- $2.10^{-2}$mol/l (2,7mg/ml) de S-méthyl, N-méthyl dithiocarbazate, et
- 0,1 à 0,3ml d'une solution aqueuse contenant $1,8.10^{-3}$mol/l de chlorure d'étain (II) dihydraté et $5,6.10^{-2}$ mol/l de pyrophosphate de sodium.

On effectue la réaction à la température ambiante pendant 30min. On obtient ainsi un produit contenant un complexe nitruro du technétium-99m.

**<u>Exemples 2 à 13.</u>**

On suit le même mode opératoire que dans l'exemple 1 pour préparer des produits contenant un complexe nitruro de technétium en utilisant le même ligand azoté, le même composé oxygéné de technétium et comme agent réducteur du chlorure d'étain avec un agent complexant. Dans tous les exemples, il s'agit de $SnCl_2$, $2H_2O$.

Les réactifs utilisés, leurs quantités et les conditions réactionnelles sont donnés dans le tableau 1 annexé.

Dans l'exemple 10 où l'on utilise comme agent complexant du gluconate de sodium, il n'est pas nécessaire d'ajouter de tampon phosphate pour ajuster le pH.

Les produits obtenus dans ces exemples sont des produits intermédiaires contenant un complexe de technétium 99m, qui pourront être utilisés ensuite pour la préparation de produits radiopharmaceutiques à base de $^{99m}$Tc.

**<u>Exemples 14 à 17.</u>**

Dans ces exemples, on suit le même mode opératoire que dans l'exemple 1 et l'on utilise le même composé oxygéné de métal de transition, le même ligand azoté et un tampon phosphate, mais dans ce cas, l'agent réducteur est constitué par un sel d'étain qui reste à l'état ionique dans la solution aqueuse sans adjonction d'agent complexant.

Les réactifs et les conditions de réaction utilisés dans ces exemples sont donnés dans le tableau 1.

**<u>Exemples 18 à 23.</u>**

Dans ces exemples, on suit le même mode opératoire que dans l'exemple 1, mais on utilise différents ligands azotés et éventuellement d'autres additifs que le tampon phosphate, avec le même composé oxygéné de technétium et un agent réducteur constitué par du chlorure d'étain $SnCl_2$, $2H_2O$ associé à du pyrophosphate de sodium.

Les réactifs utilisés, leur quantité et les conditions de réaction sont donnés dans le tableau 1.

Au vu de ce tableau, on constate que la nature de l'additif ajouté pour ajuster le pH dépend en particulier du ligand azoté utilisé.

**Exemple 24.**

Dans cet exemple, on suit le même mode opératoire que dans l'exemple 1, mais on utilise comme agent réducteur 0,2ml d'une solution contenant $6.10^{-2}$mol/l de dithionite de sodium dans de l'eau.

Les conditions de réaction et les quantités de réactif utilisées sont données dans le tableau 1.

**Exemples 25 à 46.**

Dans ces exemples, on utilise les produits préparés dans les exemples 1 à 24 pour former dans une seconde étape des complexes nitruro de technétium-99m avec du diéthyldithiocarbamate de sodium, ce qui conduit à des produits radiopharmaceutiques présentant notamment un tropisme cardiaque.

Lorsque l'agent réducteur utilisé dans la première étape est de l'étain, il est préférable de réaliser la seconde étape en présence d'un agent complexant ayant un pouvoir complexant vis-à-vis de l'étain supérieur à celui du dithiocarbamate car sinon il y aurait une précipitation de dithiocarbamate d'étain. Aussi, bien que ce précipité n'altère pas la pureté radiochimique du complexe nitruro de $^{99m}$Tc, on ajoute 0,2ml d'une solution d'un agent complexant constitué par l'acide diaminopropane-1,2 N,N,N',N'-tétracétique en solution dans de l'eau à une concentration de 50mg/ml.

Cet acide peut remplacer les agents complexants de l'étain utilisés dans la première étape tels que le pyrophosphate, le gluconate, etc.

Dans l'exemple 25, on procède de la façon suivante :

On ajoute au flacon contenant le produit préparé dans l'exemple 1, 0,2ml d'une solution contenant 0,33mol/l d'acide diaminopropane-1,2 N,N,N',N'-tétracétique dans de l'eau, puis 0,5ml d'une solution contenant $4.10^{-2}$mol/l de diéthyldithiocarbamate de sodium dans de l'eau, à un pH de 7,5 à 11.

On effectue la réaction pendant 15min à 100°C.

La pureté radiochimique du complexe obtenu est testée en effectuant une chromatographie sur couche mince (CCM) utilisant un gel de silice et un mélange de dichlorométhane et de toluène dans le rapport volumique 1:1 comme solvant.

Le complexe obtenu a un Rf de 0,4 à 0,5.

La pureté radiochimique est supérieure à 93%.

Une comparaison avec un échantillon de nitruro-bis(diéthyldithiocarbamato)$^{99}$Tc obtenu selon la méthode de Baldas décrite dans la référence J. Chem. Soc. Dalton Trans. 1981, p. 1798-1801 montre qu'il s'agit bien du même complexe nitruro de technétium.

Dans les exemples 26 à 46, on suit le même mode opératoire que dans l'exemple 25, en utilisant d'autres produits que celui de l'exemple 1 et éventuellement des conditions de réaction différentes.

Les conditions de réaction, les réactifs utilisés et leurs quantités sont donnés dans le tableau 2.

Dans ce tableau, on a également indiqué la pureté radiochimique du complexe obtenu.

Au vu de ce tableau, on constate que le procédé de l'invention permet d'obtenir aisément le nitruro-bis(diéthyldithiocarbamato)$^{99m}$Tc avec un degré de pureté radiochimique élevé.

On remarque que dans l'exemple 31, il n'est pas nécessaire d'ajouter d'agent complexant car le produit préparé dans l'exemple 8 contient déjà de l'acide diaminopropane-1,2 N,N,N',N'-tétracétique.

**Exemple 46.**

Dans cet exemple, on prépare directement un produit radiopharmaceutique en mélangeant simultanément le ligand azoté, l'agent réducteur et le deuxième ligand.

Dans ce cas, on suit le même mode opératoire que dans l'exemple opératoire que dans l'exemple 1 et on utilise les mêmes quantités de réactifs, mais on introduit de plus dans le flacon 0,5ml d'une solution à $4.10^{-2}$ mol/l de diéthyldithiocarbamate. Le pH est compris entre 7,5 et 8,5 et on laisse la réaction se poursuivre pendant 30 minutes à 100°C.

La pureté radiochimique du complexe obtenu est de 92%.

**Exemples 47 à 69.**

On suit le même mode opératoire que dans l'exemple 1 pour préparer des produits contenant un complexe nitruro de technétium en utilisant comme premier ligand azoté les ligands mentionnés dans le tableau 3 annexé,

comme agent réducteur $SnCl_2$, $2H_2O$ avec un agent complexant constitué par l'acide diaminopropane-1,2 N,N,N',N'-tétracétique et comme composé oxygéné de technétium une solution stérile de pertechnétate de sodium (Tc-99m) correspondant à une radioactivité allant de 18 MBq à 18,5 GBq (0,5-500 mCi).

Les réactifs utilisés, leurs quantités et les conditions réactionnelles sont donnés dans le tableau 3 annexé.

Au vu de ce tableau, on remarque qu'il est possible d'effectuer la réaction à la température ambiante avec de nombreux ligands azotés.

Les produits obtenus dans ces exemples sont des produits intermédiaires contenant un complexe de technétium 99m, qui pourront être utilisés ensuite pour la préparation de produits radiopharmaceutiques à base de $^{99m}Tc$.

**Exemples 70 à 97.**

Dans ces exemples, on utilise les produits préparés dans les exemples 47 à 69 et le produit préparé dans l'exemple 8 pour former dans une seconde étape des complexes nitruro de technétium-99m avec un second ligand constitué par du diéthyldithiocarbamate de sodium ou par les différents ligands donnés dans le tableau 4 annexé. On obtient ainsi des produits radiopharmaceutiques.

Dans ces exemples, on suit le même mode opératoire que dans l'exemple 31 en utilisant les réactifs et les conditions réactionnelles données dans le tableau 4 annexé.

Dans ce tableau, on a également indiqué la pureté radiochimique du complexe obtenu qui a été déterminée par une méthode chromatographique par CCM analogue à la méthode de l'exemple 25.

Au vu de ce tableau, on constate que le procédé de l'invention permet d'obtenir aisément divers complexes nitruro de technétium utilisables comme produits radiopharmaceutiques, avec un degré de pureté radiochimique élevé, même lorsque l'on opère à la température ambiante. De plus, on peut opérer à un pH physiologique, ce qui est avantageux pour les applications dans les domaines du diagnostic etde la thérapie.

Le procédé de l'invention est donc très intéressant puisqu'il permet d'opérer dans des conditions douces (température ambiante et/ou pH physiologique) lorsque l'on choisit des ligands appropriés.

**TABLEAU 1**

| Ex. | 1er ligand azoté | Agent réducteur | Autre additif | Composé oxygéné de M | Conditions de réaction |
|---|---|---|---|---|---|
| 1 | (0,1-0,5ml) S-méthyl, N-méthyl dithiocarbazate ($2.10^{-2}$ mol/l) 2,7mg/ml | (0,1-0,3ml) $SnCl_2$, $2H_2O$ ($1,8.10^{-3}$ mol/l) pyrophosphate de sodium ($5,6.10^{-2}$mol/l) | (1ml) tampon phosphate ( 0,1-0,5mol/l) (pH:7,4-8) | (0,5-3ml) pertechnétate de Na ($^{99m}Tc$ ) 18MBq à 3,7GBq (0,5-100mCi) | température ambiante, 30min |
| 2 | " | " | " | " | 100°C, 20min |
| 3 | " | " | " | " | 100°C, 30min |
| 4 | " | (0,1-0,3ml) $SnCl_2$ ($1,8.10^{-3}$ mol/l) glucoheptonate de sodium (0,18mol/l) | " | " | 100°C, 30min |

../

**TABLEAU 1** (suite)

| Ex. | 1er Ligand azoté | Agent réducteur | Autre additif | Composé oxygéné de M | Conditions de réaction |
|---|---|---|---|---|---|
| 5 | " | (0,1-0,3mL) $SnCl_2$ ($5.10^{-4}$ mol/l) diéthylène triamino pentacétate de sodium (DTPA) ($6.10^{-3}$mol/l) | " | " | température ambiante, 30min |
| 6 | " | " | " | " | 100°C, 30min |
| 7 | " | (0,1-0,3mL) $SnCl_2$, ($5.10^{-4}$ mol/l) éthylène diaminotétracétate de Na (EDTA) ($6.10^{-3}$mol/l) | " | " | 100°C, 30min |

../

**TABLEAU 1** (suite)

| Ex. | 1er ligand azoté | Agent réducteur | Autre additif | Composé oxygéné de M | Conditions de réaction |
|---|---|---|---|---|---|
| 8 | " | (0,2-0,5ml) $SnCl_2$ ($4.10^{-3}$ mol/l) diamino - 1,2 propane N,N,N',N' tétracétate de Na (0,16mol/l) | " | " | 100°C, 30min |
| 9 | " | (0,1-0,5ml) $SnCl_2$ ($3.10^{-4}$ mol/l) glucoheptonate de Na (0,18mol/l) | " | " | 100°C, 30min |
| 10 | " | (0,1-0,5ml) $SnCl_2$ ($1,7.10^{-3}$ mol/l) gluconate de Na (0,1mol/l) | aucun | " | 100°C, 30min |
| 11 | " | (0,1-0,2ml) $SnCl_2$ ($5,5.10^{-4}$ mol/l) méthylène diphosphonate de Na ($7.10^{-3}$mol/l) | (1ml) tampon phosphate (0,1-0,5mol/l) pH:7,4-8 | " | température ambiante, 30min |
| 12 | " | (0,1-0,5ml) $SnCl_2$ ($7.10^{-4}$ mol/l) hydroxyméthylène diphosphonate de sodium ($5.10^{-3}$mol/l) | " | " | 100°C, 20min |

**TABLEAU 1** (suite)

| Ex. | 1er ligand azoté | Agent réducteur | Autre additif | Composé oxygéné de M | Conditions de réaction |
|---|---|---|---|---|---|
| 13 | " | (0,1-0,5ml) $SnCl_2$ ($1.10^{-3}$ mol/l) citrate de Na (0,1mol/l) | " | " | 100°C, 30min |
| 14 | " | (0,1-0,5ml) tartrate de Sn ($7.10^{-4}$mol/l) | " | " | température ambiante 30min |
| 15 | " | " | " | " | 100°C, 30min |
| 16 | " | (0,1-0,5ml) oxalate de Sn (solution aqueuse saturée) | " | " | température ambiante 30min |
| 17 | " | " | " | " | 100°C, 20min |

../

**TABLEAU 1** (suite)

| Ex. | 1er ligand azoté | Agent réducteur | Autre additif | Composé oxygéné de M | Conditions de réaction |
|---|---|---|---|---|---|
| 18 | (0,1-0,5ml) azoture de sodium ($5.10^{-2}$ mol/l) | (0,1-0,3ml) $SnCl_2$ ($1,8.10^{-3}$mol/l) pyrophosphate de Na ($5,6.10^{-2}$mol/l) | " | " | 100°C, 30min |
| 19 | (0,1-0,5ml) dichlorhydrate d'hydrazine (0,1mol/l) | " | (0,1ml) HCl 1N | " | " |
| 20 | " | " | (1ml) tampon PIPES (acide pipérazine diéthane sulfonique-1,4 (0,1mol/l pH:5,0) | " | " |
| 21 | (0,2ml) acéthydrazide (0,13mol/l) | " | " | " | " |

../

**TABLEAU 1** (suite)

| Ex. | 1er ligand azoté | Agent réducteur | Autre additif | Composé oxygéné de M | Conditions de réaction |
|---|---|---|---|---|---|
| 22 | (0,2ml) semicarbazide (0,1mol/l) | " | " | " | " |
| 23 | (0,4ml) 2-méthylthio semi carbazide ($5.10^{-2}$mol/l) | " | " | " | " |
| 24 | (0,1-0,5ml) S-méthyl,N-méthyl dithiocarbazate ($2.10^{-2}$mol/l) | (0,2ml) dithionite de Na ($6.10^{-2}$mol/l) | (1ml) tampon phosphate (0,1-0,5mol/l pH:7,4-8) | " | 100°C, 30min |

**TABLEAU 2**

| Ex. | Produit | Second ligand | Agent complexant l'étain | Conditions de réaction | Pureté radiochimique |
|---|---|---|---|---|---|
| 25 | Ex.1 | (0,5ml) diéthyldithio carbamate de Na ($4.10^{-2}$mol/l) pH 7,5-11 | (0,2ml) acide diamino propane-1,2 N,N,N',N' tétra-cétique (0,33mol/l) | 100°C, 15min | > 93% |
| 26 | Ex.3 | " | " | température ambiante, 15min | > 98% |
| 27 | Ex. 4 | " | " | " | " |
| 28 | Ex. 5 | " | " | 100°C, 15min | 96% |
| 29 | Ex. 6 | " | " | température ambiante, 15min | 97% |
| 30 | Ex.7 | " | " | " | 95% |

../

**TABLEAU** 2 (suite)

| Ex. | Produit | Second ligand | Agent complexant l'étain | Conditions de réaction | Pureté radiochimique |
|---|---|---|---|---|---|
| 31 | Ex. 8 | " | aucun | " | 96% |
| 32 | Ex. 9 | " | (0,2ml) acide diamino propane-1,2 N,N,N',N' tétra-cétique (0,33mol/l) | " | ≥ 97% |
| 33 | Ex. 10 | " | " | " | > 92% |
| 34 | Ex. 11 | " | " | 100°C, 15min | > 94% |
| 35 | Ex. 12 | " | " | Température ambiante 15min | > 92% |
| 36 | Ex. 13 | " | " | " | > 98% |
| 37 | Ex. 14 | " | " | 100°C, 15min | 95% |

../

**TABLEAU 2** (suite)

| Ex. | Produit | Second ligand | Agent complexant l'étain | Conditions de réaction | Pureté radiochimique |
|---|---|---|---|---|---|
| 38 | Ex. 15 | " | " | Température ambiante, 15min | 98% |
| 39 | Ex. 16 | " | " | " | 98% |
| 40 | Ex. 18 | " | " | " | 95% |
| 41 | Ex. 19 | " | " | " | 93% |
| 42 | Ex. 20 | " | " | " | 92% |
| 43 | Ex. 21 | " | " | 100°C, 15min | 84% |
| 44 | Ex. 22 | " | " | " | 88% |
| 45 | Ex. 23 | " | " | Température ambiante 15min | 97% |
| 46 | Ex. 24 | " | aucun | 100°C,15min | 91% |

## TABLEAU 3

| Ex. | 1er ligand azoté | Agent réducteur | Autre additif | Composé oxygéné de M | Conditions de réaction |
|---|---|---|---|---|---|
| 47 | 1 ml tert-butyl carbazate ($4x10^{-2}$ mol/l) 5,3 mg/ml 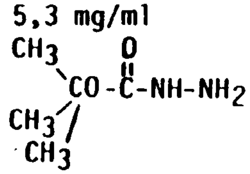 | 0,5 ml $SnCl_2$, $2H_2O$ ($9x10^{-4}$ mol/l acide diamino propane-1,2 N,N,N',N' tétraacétique ($6,5x10^{-2}$ mol/l) | 1ml tampon phosphate (0,1-0,5 mol/l) (pH=7,4-8) | (0,5-3 ml) pertechnétate de sodium ($^{99m}Tc$) 18MBq à 3,7GBq (0,5-100 mCi) | 100°C 30 min. |
| 48 | 1ml benzoyl hydrazide ($10^{-1}$ mol/l) 13,6 mg/ml 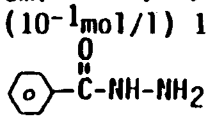 | " | " | " | 100°C 30 min. |
| 49 | 0,2-0,5ml Réactif P de Girard ($2x10^{-2}$ mol/l) 37,6mg/ml 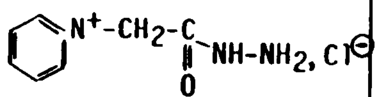 | " | " | " | 100°C 30 min. |

./..

TABLEAU 3 (Suite)

| Ex. | 1er ligand azoté | Agent réducteur | Autre additif | Composé oxygéné de M | Conditions de réaction |
|---|---|---|---|---|---|
| 50 | 0,2-0,5 ml méthyl hydrazine carboxylate ($2x10^{-2}$ mol/l) 18 mg/cc<br>$CH_3$-C-NH-$NH_2$<br>‖<br>O | " | " | " | 100°C<br>30 min. |
| 51 | 0,1-1 ml formyl hydrazide ($10^{-1}$ mol/l)<br>6 mg/ml<br>H-C-NH-$NH_2$<br>‖<br>O | " | " | " | 100°C<br>30 min. |
| 52 | " | " | " | " | 30 min.<br>Temp. ambiante |
| 53 | 0,1-1 ml oxamyl hydrazide ($10^{-1}$ mol/l)<br>10,3 mg/ml<br>$H_2N$-C-C-NH-$NH_2$<br>‖ ‖<br>O O | " | " | " | 100°C<br>30 min. |

./..

TABLEAU 3 (Suite)

| Ex. | 1er ligand azoté | Agent réducteur | Autre additif | Composé oxygéné de M | Conditions de réaction |
|---|---|---|---|---|---|
| 54 | 0,1-1 ml cyanoacéto-hydrazide ($10^{-1}$ mol/l) 9,9 mg/ml 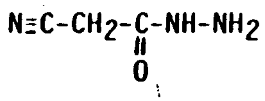 | " | " | " | 100°C 30 min. |
| 55 | 0,1-1 ml succinyl dihydrazide ($10^{-1}$ mol/l) 14,6 mg/ml 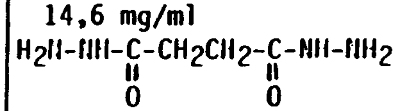 | " | " | " | Temp. ambiante 30 min. |
| 56 | " | " | " | " | 100°C 30 min. |
| 57 | 0,1-1 ml 4-hydroxy benzoyl hydrazide ($10^{-1}$ mol/l) 25,2 mg/ml 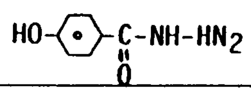 | " | " | " | 100°C 30 min. |

./..

TABLEAU 3 (Suite)

| Ex. | 1er ligand azoté | Agent réducteur | Autre additif | Composé oxygéné de M | Conditions de réaction |
|---|---|---|---|---|---|
| 58 | 0,1-1 ml 4-amino benzoyl hydrazide ($10^{-1}$ mol/l) 15,1 mg/mol 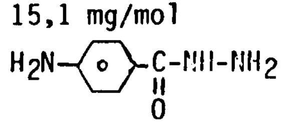 | " | " | " | Temp. ambiante 30 min. |
| 59 | " | " | " | " | 100°C 30 min. |
| 60 | 0,1-1 ml meta-anisyl hydrazide ($10^{-1}$ mol/l) 16,6 mg/ml 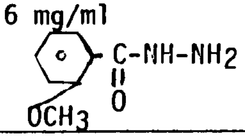 | " | " | " | 100°C 30 min. |
| 61 | 0,1-1 ml benzène sulfonyl hydrazide ($10^{-1}$ mol/l) 17,2 mg/ml 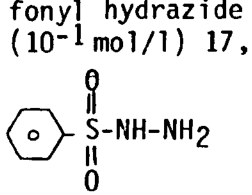 | " | " | " | Temp. ambiante 30 min. |

./..

TABLEAU 3 (Suite)

| Ex. | 1er ligand azoté | Agent réducteur | Autre additif | Composé oxygéné de M | Conditions de réaction |
|---|---|---|---|---|---|
| 62 | " | " | " | " | 100°C 30 min. |
| 63 | 0,1-1 ml para-toluène sulfonyl hydrazide ($10^{-1}$ mol/l) 18,6 mg/ml 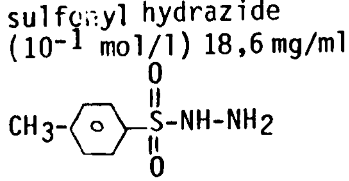 | " | " | " | Temp. ambiante 30 min. |
| 64 | " | " | " | " | 100°C 30 min. |
| 65 | 0,1-1 ml 2,4,5 trichlorobenzène sulfonyl hydrazide ($10^{-1}$ mol/l) 27,5 mg/ml 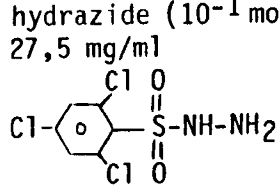 | " | " | " | Temp. ambiante 30 min. |
| 66 | " | " | " | " | 100°C 30 min. |

./..

TABLEAU 3 (Suite)

| Ex. | 1er ligand azoté | Agent réducteur | Autre additif | Composé oxygéné de M | Conditions de réaction |
|---|---|---|---|---|---|
| 67 | 0,1-1 ml 2,4,6 triméthyl benzène sulfonyl hydrazide ($10^{-1}$ mol/l) 21,4 mg/ml 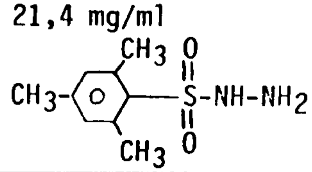 | " | " | " | Temp. ambiante 30 min. |
| 68 | " | " | " | " | 100°C 30 min. |
| 69 | 0,1 ml de S-méthyl, N-méthyl dithiocarbazate (0,2 mg/ml) | 0,2 ml $SnCl_2$, $2H_2O$ ($4.10^{-3}$ mol/l) diamino-1,2 propane N,N,N',N'-tétracétate de sodium (0,16 mol/l) | " | " | Temp. ambiante 30 min. |

TABLEAU 4

| Ex. | Produit | Second ligand | Conditions de réaction | Pureté radiochimique |
|---|---|---|---|---|
| 70 | Ex. 47 | 0,5 ml de diéthyl dithiocarbamate de Na ($4x10^{-2}$ mol/l) pH = 7,5-11 | Temp. ambiante 15 min. | 96 % |
| 71 | Ex. 48 | " | " | 94 % |
| 72 | Ex. 49 | " | " | 98 % |
| 73 | Ex. 50 | " | " | 98 % |
| 74 | Ex. 51 | " | " | 90 % |
| 75 | Ex. 52 | " | " | 96 % |
| 76 | Ex. 53 | " | " | 95 % |
| 77 | Ex. 54 | " | " | 90 % |
| 78 | Ex. 55 | " | " | 98 % |
| 79 | Ex. 56 | " | " | 96 % |

./..

**TABLEAU 4** (Suite)

| Ex. | Produit | Second ligand | Conditions de réaction | Pureté radiochimique |
|---|---|---|---|---|
| 80 | Ex. 57 | " | " | 94 % |
| 81 | Ex. 58 | " | " | 90 % |
| 82 | Ex. 59 | 0,5 ml diéthyl dithiocarbamate de Na ($4x10^{-2}$ mol/l) pH = 7,5-11 | Temp. ambiante 15 min. | 85 % |
| 83 | Ex. 60 | " | " | 90 % |
| 84 | Ex. 61 | " | " | 92 % |
| 85 | Ex. 62 | " | " | 96 % |
| 86 | Ex. 63 | " | " | 91 % |
| 87 | Ex. 64 | " | " | 96 % |
| 88 | Ex. 65 | " | " | 96 % |
| 89 | Ex. 66 | " | " | 97 % |
| 90 | Ex. 67 | " | " | 91 % |

./..

**TABLEAU 4** (Suite)

| Ex. | Produit | Second ligand | Conditions de réaction | Pureté radiochimique |
|---|---|---|---|---|
| 91 | Ex. 68 | " | " | 96 % |
| 92 | Ex. 69 | " | " | 96 % |
| 93 | Ex. 8 | 1 ml 2-thioquinoléïne 1 mg/ml<br>pH = 7,80<br>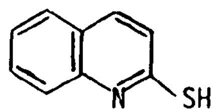 | 100°C<br>30 min. | > 90 % |
| 94 | " | 1 ml (D.L) pénicillamine 10 mg/ml<br>pH = 7,80<br>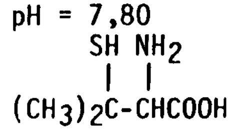 | Temp. ambiante<br>30 min. | 95 % |
| 95 | " | 1 ml éthyl dithiocarboxylate de sodium<br>10 mg/ml pH = 3,5<br>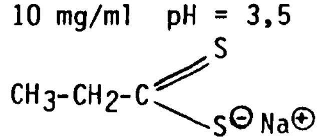 | Temp. ambiante<br>30 min. | 85 % |

./..

**TABLEAU 4** (Suite)

| Ex. | Produit | Second ligand | Conditions de réaction | Pureté radiochimique |
|---|---|---|---|---|
| 96 | " | 1 ml isopropyl xanthate de sodium<br>10 mg/ml pH = 7,80<br>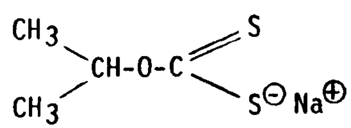 | Temp. ambiante<br>30 min. | > 90 % |
| 97 | Ex. 8 | 1 ml diéthyl dithiophosphinate de sodium<br>10 mg/ml pH = 5<br>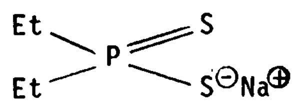 | Temp. ambiante<br>30 min. | > 90 % |

## Revendications

1. Procédé de préparation d'un produit comprenant un complexe nitruro d'un métal de transition comportant une partie M≡N avec M représentant le technétium ou le rhénium, caractérisé en ce que l'on fait réagir en solution un composé oxygéné du métal de transition M avec
   1°) un premier ligand azoté constitué soit par un azoture d'ammonium ou de métal pharmaceutiquement acceptable, soit par un composé azoté comportant un motif ⟩N-N⟨ dans lequel les N sont reliés à des atomes d'hydrogène et/ou à des groupements organiques monovalents ou dans lequel l'un des N est relié à l'atome de carbone d'un groupement organique bivalent par l'intermédiaire d'une double liaison et l'autre N est relié à des atomes d'hydrogène et/ou à des groupements organiques monovalents, et
   2°) un agent réducteur constitué, soit par du dithionite d'ammonium ou d'un métal pharmaceutiquement acceptable, soit par de l'étain (II) présent sous forme ionique dans la solution.

2. Procédé de préparation d'un produit comprenant un complexe nitruro d'un métal de transition comportant une partie M≡N dans laquelle M représente le technétium ou le rhénium, caractérisé en ce que l'on fait réagir en solution un composé oxygéné du métal de transition M avec :
   1°) un premier ligand azoté constitué, soit par un azoture d'ammonium ou de métal pharmaceutiquement acceptable, soit par un composé azoté comportant un motif ⟩N-N⟨ dans lequel les N sont reliés à des atomes d'hydrogène et/ou à des groupements organiques monovalents ou dans lequel l'un des N est relié à l'atome de carbone d'un groupe organique bivalent par l'intermédiaire d'une double liaison et l'autre N est relié à des atomes d'hydrogène et/ou à des groupements organiques monovalents ;
   2°) un agent réducteur constitué soit par un dithionite d'ammonium ou de métal pharmaceutiquement acceptable, soit par de l'étain II présent sous forme ionique dans la solution ; et
   3°) un deuxième ligand organique à groupement nucléophile, un anticorps monoclonal, un fragment d'anticorps, une protéine ou un peptide.

3. Procédé selon la revendication 2, caractérisé en ce que, dans une première étape, on fait réagir le composé oxygéné du métal de transition avec le premier ligand et l'agent réducteur, et dans une deuxième étape, on fait réagir le produit obtenu à la suite de la première étape avec le deuxième ligand, l'anticorps monoclonal ou le fragment d'anticorps.

4. Procédé selon la revendication 2, caractérisé en ce que l'on fait réagir simultanément le composé oxygéné du métal de transition avec le premier ligand azoté, l'agent réducteur et le deuxième ligand ou l'anticorps monoclonal ou le fragment d'anticorps.

5. Procédé selon l'une quelconque des revendications 1 à 4, caractérisé en ce que l'agent réducteur est l'étain (II) et en ce qu'il est introduit sous forme ionique dans la solution en ajoutant un sel d'étain (II) et un agent complexant à une solution du composé oxygéné de métal de transition et du premier ligand azoté.

6. Procédé selon la revendication 5, caractérisé en ce que le sel d'étain (II) est le chlorure d'étain (II).

7. Procédé selon l'une quelconque des revendications 5 et 6, caractérisé en ce que l'agent complexant est choisi dans le groupe comprenant les pyrophosphates de métal alcalin ou d'ammonium, les glucoheptonates de métal alcalin ou d'ammonium, les diéthylène triamino pentacétates de métal alcalin ou d'ammonium, les éthylènediaminotétracétates de métal alcalin ou d'ammonium, les diamino-1,2 propane N,N,N',N'-tétracétates de métal alcalin ou d'ammonium, les gluconates de métal alcalin ou d'ammonium, les méthylène diphosphonates de métal alcalin ou d'ammonium, les hydroxyméthylène diphosphonates de métal alcalin ou d'ammonium, et les citrates de métal alcalin ou d'ammonium.

8. Procédé selon l'une quelconque des revendications 1 à 3, caractérisé en ce que l'agent réducteur est l'étain (II) et en ce qu'il est introduit dans la solution sous forme de sel d'étain (II).

9. Procédé selon la revendication 8, caractérisé en ce que le sel d'étain (II) est le tartrate ou l'oxalate d'étain (II).

10. Procédé selon l'une quelconque des revendications 1 à 3, caractérisé en ce que l'agent réducteur est le dithionite de sodium.

11. Procédé selon l'une quelconque des revendications 1 à 10, caractérisé en ce que la solution est une solution aqueuse.

**12.** Procédé selon la revendication 11, caractérisé en ce que la solution aqueuse a un pH allant de 7 à 8.

**13.** Procédé selon l'une quelconque des revendications 1 à 12, caractérisé en ce que le premier ligand azoté est le S-méthyl, N-méthyl dithiocarbazate.

**14.** Procédé selon l'une quelconque des revendications 1 à 11, caractérisé en ce que le premier ligand azoté est choisi dans le groupe comprenant l'azoture de sodium, l'hydrazine, l'acéthydrazide, le semicarbazide et le méthyl-2 thiosemicarbazide.

**15.** Procédé selon l'une quelconque des revendications 1 à 11, caractérisé en ce que le premier ligand azoté répond à la formule

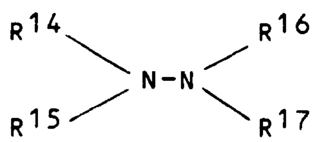

dans laquelle $R^{14}$, $R^{15}$ et $R^{16}$ qui peuvent être identiques ou différents, représentent un atome d'hydrogène, un radical alkyle, un radical aryle, un radical alcoxy, un radical alkyle substitué par au moins un groupement choisi parmi les radicaux hydroxy, carboxy, amino, amido et mercapto ou un radical aryle substitué par au moins un groupement choisi parmi les atomes d'halogène et les radicaux alcoxy, hydroxy, amino, mercapto et amino substitué par au moins un radical alkyle,

$R^{17}$ représente un radical répondant aux formules

$$-SO_2R^{18} \text{ ou } -CO\,R^{19}$$

dans lesquelles $R^{18}$ est un groupe phényle non substitué ou substitué par au moins un substituant choisi parmi les atomes d'halogène et les radicaux alkyle, et

$R^{19}$ est un atome d'hydrogène, $-NH_2$ ou un radical choisi parmi les radicaux alkyle, les radicaux alkyle substitués par au moins un groupement choisi parmi les groupements cyano, pyridyle et $-CO-NH-NH_2$, le radical phényle, le radical phényle substitué par au moins un substituant choisi parmi -OH, $NH_2$ et les radicaux alkyle et alcoxy.

**16.** Procédé selon la revendication 15, caractérisé en ce que $R^{14}$, $R^{15}$ et $R^{16}$ sont des atomes d'hydrogène, $R^{17}$ représente le radical de formule $SO_2R^{18}$ avec $R^{18}$ ayant la signification donnée dans la revendication 15, ou le radical de formule COH, $CO-CH_2-CH_2-CO-NH-NH_2$ ou

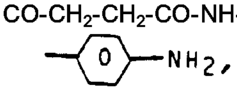

et en ce que l'on effectue la réaction à la température ambiante.

**17.** Procédé selon la revendication 16, caractérisé en ce que $R^{17}$ représente $SO_2R^{18}$ avec $R^{18}$ étant le radical phényle, le radical p-méthyl phényle, le radical trichloro-1,3,5 phényle ou le radical triméthyl-1,3,5 phényle.

**18.** Procédé selon l'une quelconque des revendications 2 à 17, caractérisé en ce que le deuxième ligand est le diéthyldithiocarbamate de sodium.

**19.** Procédé selon l'une quelconque des revendications 2 à 17, caractérisé en ce que le deuxième ligand est choisi parmi la thioquinoléine, la pénicillamine, l'éthyldithiocarboxylate de sodium, l'isopropylxanthate de sodium et le diéthydithiophosphinate de sodium.

**20.** Procédé selon l'une quelconque des revendications 1 à 19, caractérisé en ce que le composé oxygéné du métal de transition est un pertechnétate -99m de métal alcalin ou d'ammonium.

**21.** Procédé selon l'une quelconque des revendications 1 à 19, caractérisé en ce que le composé oxygéné du métal de transition est du perrhénate -186 ou -188 de métal alcalin ou d'ammonium.

22. Trousse pour la préparation d'un complexe nitruro de métal de transition par mise en oeuvre du procédé selon l'une quelconque des revendications 2 à 21, caractérisée en ce qu'elle comprend :
- un premier flacon contenant le premier ligand azoté et l'agent réducteur, et
- un deuxième flacon contenant un deuxième ligand organique à groupement nucléophile, un anticorps monoclonal, un fragment d'anticorps, une protéine ou un peptide.

## Claims

1. Process for the preparation of a product incorporating a nitride complex of a transition metal having a part M ≡ N with M representing technetium or rhenium transition metal, characterized in that reaction takes place in solution of an oxygen compound of the transition metal M with
1°) a first nitrogen ligand constituted either by a pharmaceutically acceptable metal or ammonium nitride, or by a nitrogen compound having a
$>N-N<$
unit, in which the N's are connected to hydrogen atoms and/or monovalent organic groups or in which one of the N's is connected to the carbon atom of a divalent organic group by means of a double bond and the other N is connected to hydrogen atoms and/or monovalent organic groups and
2°) a reducing agent constituted either by a pharmaceutically acceptable metal or ammonium dithionite, or by tin (II) present in ionic form in the solution.

2. Process for the preparation of a product incorporating a nitride complex of a transition metal having a part M ≡ N, in which M represents technetium or rhenium, characterized in that reaction takes place in solution of an oxygen compound of the transition metal M with:
1°) a first nitrogen ligand constituted either by a pharmaceutically acceptable metal or ammonium nitride, or by a nitrogen compound having a
$>N-N<$
unit, in which the N's are connected to hydrogen atoms and/or to monovalent organic groups, or in which one of the N's is connected to the carbon atom of a divalent organic group by means of a double bond and the other N is connected to hydrogen atoms and/or to monovalent organic groups,
2°) a reducing agent constituted either by a pharmaceutically acceptable metal or ammonium dithionite or by tin (II) present in ionic form in the solution and
3°) a second organic ligand having a nucleophilic group, a monoclonal antibody, an antibody fragment, a protein or a peptide.

3. Process according to claim 2, characterized in that, in a first stage, the oxygen compound of the transition metal is reacted with the first ligand and the reducing agent, and in a second stage, the product obtained at the end of the first stage is reacted with the second ligand, the monoclonal antibody or the antibody fragment.

4. Process according to claim 2, characterized in that simultaneous reaction takes place between the oxygen compound of the transition metal and the first nitrogen ligand, the reducing agent and the second ligand or the monoclonal antibody or antibody fragment.

5. Process according to any one of the claims 1 to 4, characterized in that the reducing agent is tin (II) and in that it is introduced in ionic form into the solution by adding a tin (II) salt and a complexing agent to a solution of the transition metal oxygen compound and the first nitrogen ligand.

6. Process according to claim 5 characterized in that the tin (II) salt is tin (II) chloride.

7. Process according to either of the claims 5 and 6, characterized in that the complexing agent is chosen from within the group including ammonium or alkali metal pyrophosphates, ammonium or alkali metal glucoheptonates, ammonium or alkali metal diethylene triaminopentaacetates, ammonium or alkali metal ethylene diaminotetraacetates, ammonium or alkali metal 1,2-diamino-N,N,N',N'-propane tetraacetates, ammonium or alkali metal gluconates, ammonium or alkali metal methylene diphosphonates, ammonium or alkali metal hydroxymethylene diphosphonates and ammonium or alkali metal citrates.

8. Process according to any one of the claims 1 to 3, characterized in that the reducing agent is tin (II) and in that it is introduced into the solution in the form of a tin (II) salt.

**9.** Process according to claim 8, characterized in that the tin (II) salt is tin (II) oxalate or tartrate.

**10.** Process according to any one of the claims 1 to 3, characterized in that the reducing agent is sodium dithionite.

**11.** Process according to any one of the claims 1 to 10, characterized in that the solution is an aqueous solution.

**12.** Process according to claim 11, characterized in that the aqueous solution has a pH of 7 to 8.

**13.** Process according to any one of the claims 1 to 12 characterized in that the first nitrogen ligand is S-methyl, N-methyl dithiocarbazate.

**14.** Process according to any one of the claims 1 to 11, characterized in that the first nitrogen ligand is chosen from within the group including sodium nitride, hydrazine, acetohydrazide, semicarbazide and 2-methyl thiosemicarbazide.

**15.** Process according to any one of the claims 1 to 11, characterized in that the first nitrogen ligand is in accordance with the formula:

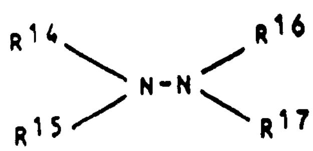

in which $R^{14}$, $R^{15}$ and $R^{16}$, which can be the same or different, represent a hydrogen atom, a alkyl radical, an aryl radical, an alkoxy radical, an alkyl radical substituted by at least one group chosen from among hydroxy, carboxy, amino, amido and mercapto radicals or an aryl radical substituted by at least one group chosen from among halogen atoms and alkoxy, hydroxy, amino and mercapto radicals and amino radicals substituted by at least one alkyl radical, $R^{17}$ represents a radical complying with the formulas

$-SO_2R^{18}$ or $-CO\ R^{19}$

in which $R^{18}$ is a phenyl group, which is not substituted or substituted by at least one substituent chosen from among the halogen atoms and the alkyl radicals and $R^{19}$ is a hydrogen atom, $-NH_2$ or a radical chosen from among alkyl radicals, alkyl radicals substituted by at least one group chosen from among cyano, pyridyl and $-CO-NH-NH_2$ groups, the phenyl radical, the phenyl radical substituted by at least one substituent chosen from among -OH, $NH_2$ and alkyl and alkoxy radicals.

**16.** Process according to claim 15, characterized in that $R^{14}$, $R^{15}$ and $R^{16}$ are hydrogen atoms, $R^{17}$ represents the radical of formula $SO_2R^{18}$ with $R^{18}$ having the meaning given in claim 15, or the radical of formula COH, $CO-CH_2-CH_2-CO-NH-NH_2$ or

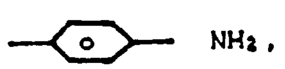

and in that the reaction is performed at ambient temperature.

**17.** Process according to claim 16, characterized in that $R^{17}$ represents $SO_2R^{18}$ with $R^{18}$ being the phenyl radical, the p-methyl phenyl radical, the 1,3,5-trichlorophenyl radical or the 1,3,5-trimethyl phenyl radical.

**18.** Process according to any one of the claims 2 to 17, characterized in that the second ligand is sodium diethyl dithiocarbamate.

**19.** Process according to any one of the claims 2 to 17, characterized in that the second ligand is chosen from among thioquinoline penicillamine sodium ethyl dithiocarboxylate, sodium isopropyl xanthate and sodium diethyl dithiophosphonate.

**20.** Process according to any one of the claims 1 to 19, characterized in that the transition metal oxygen compound is an ammonium or alkali metal pertechnetate -99m.

**21.** Process according to any one of the claims 1 to 19, characterized in that the transition metal oxygen compound is ammonium or alkali metal perrhenate -186 or -188.

22. Kit for the preparation of a transition metal nitride complex by performing the process according to any one of the claims 2 to 21, characterized in that it incorporates a first bottle containing the first nitrogen ligand and the reducing agent and a second bottle containing a second organic ligand with a nucleophilic group, a monoclonal antibody, an antibody fragment, a protein or a peptide.

## Patentansprüche

1. Verfahren zur Herstellung eines Produkts, welches einen Übergangsmetallnitridkomplex umfaßt, der einen Teil M≡N umfaßt, wobei M Technetium oder Rhenium darstellt, dadurch gekennzeichnet, daß man in Lösung eine Sauerstoffverbindung des Übergangsmetalls M reagieren läßt mit
   1°) einem ersten Stickstoffliganden, der sowohl von Ammoniumnitrid oder einem pharmazeutisch annehmbaren Metall, als auch einer Stickstoffverbindung gebildet wird, die die Grundeinheit >N-N< umfaßt, in welcher die N mit den Wasserstoffatomen und/oder den monovalenten organischen Gruppen verbunden sind oder in welcher eines der N mit dem Kohlenstoffatom einer organischen bivalenten Gruppe durch Zwischenschaltung einer Doppelbindung und das andere N mit Wasserstoffatomen und/oder monovalenten organischen Gruppen verbunden ist, und
   2°) einem Reduktionsmittel, das sowohl aus Ammoniumdithionit oder einem pharmazeutisch annehmbaren Metall, als auch dem in ionischer Form in der Lösung vorliegenden Zinn (II) gebildet wird.

2. Verfahren zur Herstellung eines Produkts, das einen Übergangsmetallnitridkomplex umfaßt, welcher einen Teil N≡M umfaßt, in welchem M Technetium oder Rhenium darstellt, dadurch gekennzeichnet, daß man in Lösung eine Sauerstoffverbindung des Übergangsmetalls M reagieren läßt mit:
   1°) einem ersten Stickstoffliganden, der sowohl aus Ammoniumnitrid oder einem pharmazeutisch annehmbaren Metall als auch einer Stickstoffverbindung gebildet wird, die eine Grundeinheit >N-N< umfaßt, in welcher die N mit Wasserstoffatomen und/oder monovalenten organischen Gruppen verbunden sind oder in welcher eines der N mit dem Kohlenstoffatom einer organischen bivalenten Gruppe durch Zwischenschaltung einer Doppelbindung verbunden ist und das andere N mit Wasserstoffatomen und/oder monovalenten organischen Gruppen verbunden ist;
   2°) einem Reduktionsmittel, das sowohl aus Ammoniumdithionit oder einem pharmazeutisch annehmbaren Metall, als auch dem in ionischer Form in der Lösung vorliegenden Zinn (II) gebildet wird; und
   3°) einem zweiten organischen Liganden mit nukleophilen Gruppen, einem monoclonalen Antikörper, einem Antikörperfragment, einem Protein oder einem Peptid.

3. Verfahren gemäß Anspruch 2, dadurch gekennzeichnet, daß man in einem ersten Schritt die Sauerstoffverbindung des Übergangsmetalls mit dem ersten Liganden und dem Reduktionsmittel reagieren läßt und in einem zweiten Schritt das in der Folge des ersten Schritts erhaltene Produkt mit dem zweiten Liganden, dem monoclonalen Antikörper oder dem Antikörperfragment reagieren läßt.

4. Verfahren gemäß Anspruch 2, dadurch gekennzeichnet, daß man gleichzeitig die Sauerstoffverbindung des Übergangsmetalls mit dem ersten Stickstoffliganden, dem Reduktionsmittel und dem zweiten Liganden oder dem monoclonalen Antikörper oder dem Antikörperfragment reagieren läßt.

5. Verfahren gemäß einem der Ansprüche 1 bis 4, dadurch gekennzeichnet, daß das Reduktionsmittel Zinn (II) ist und daß es in ionischer Form in die Lösung eingeführt wird, indem man Zinn-(II)-Salz und einen Komplexbildner zu einer Lösung der Sauerstoffverbindung des Übergangsmetalls und des ersten Stickstoffliganden hinzufügt.

6. Verfahren gemäß Anspruch 5, dadurch gekennzeichnet, daß das Zinn-(II)-Salz Zinn-(II)-Chlorid ist.

7. Verfahren gemäß einem der Ansprüche 5 und 6, dadurch gekennzeichnet, daß der Komplexbildner in der Gruppe ausgewählt wird, die die Alkalin- oder Ammonium-Metallpyrophosphate, die Alkalin- oder Ammonium-Metallglucoheptonate, die Alkalin- oder Ammonium-Metall-Diethyltriaminopentazetate, die Alkalin- oder Ammonium-Metall-Ethylendiaminotetrazetate, die Alkalin- oder Ammonium-Metall-Diamino-1,2 Propan N,N,N',N'-Tetrazetate, die Alkalin- oder Ammonium-Metallgluconate, die Alkalin- oder Ammonium-Metallmethylendiphosphate, die Alkalin- oder Ammonium-Metallhydroxymethylendiphosphonate, und die Alkalin- oder Ammonium-Metallcitrate umfaßt.

8. Verfahren gemäß einem der Ansprüche 1 bis 3, dadurch gekennzeichnet, daß das Reduktionsmittel Zinn (II) ist und daß es in die Lösung in Form eines Zinn-(II)-Salzes eingeführt wird.

9. Verfahren gemäß Anspruch 8, dadurch gekennzeichnet, daß das Zinn-(II)-Salz Zinn-(II)- Tartrat oder -Oxalat ist.

10. Verfahren gemäß einem der Ansprüche 1 bis 3, dadurch gekennzeichnet, daß das Reduktionsmittel Natriumdithionit ist.

11. Verfahren gemäß einem der Ansprüche 1 bis 10, dadurch gekennzeichnet, daß die Lösung eine wäßrige Lösung ist.

12. Verfahren gemäß Anspruch 11, dadurch gekennzeichnet, daß die wäßrige Lösung einen pH- Wert zwischen 7 und 8 hat.

13. Verfahren gemäß einem der Ansprüche 1 bis 12, dadurch gekennzeichnet, daß der erste Stickstoffligand S-Methyl, N- Methyl Dithiocarbazon ist.

14. Verfahren gemäß einem der Ansprüche 1 bis 11, dadurch gekennzeichnet, daß der erste Stickstoffligand aus der Gruppe ausgewählt wird, die Natriumnitrid, Hydrazin, Acethydrazid, Semicarbazid und Methyl-2-Thiosemicarbazid umfaßt.

15. Verfahren gemäß einem der Ansprüche 1 bis 11, dadurch gekennzeichnet, daß der erste Stickstoffligand der Formel:

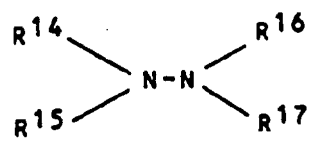

entspricht, in welcher $R^{14}$, $R^{15}$ und $R^{16}$, die gleich oder verschieden sein können, ein Wasserstoffatom, eine Alkylradikal, ein Arylradikal, ein Alkoxyradikal, ein mit mindestens einer unter den Hydroxy-, Carboxy-, Amino-, Amido- und Mercapto-Radikalen ausgewählten Gruppe substituiertes Alkylradikal oder ein mit mindestens einer unter den Halogenatomen und den Alcoxy-, Hydroxy-, Amino-, Mercapto- und mit mindestens einem Alkylradikal substituieren Amino-Radikalen ausgewählten Gruppe substituiertes Arylradikal darstellen,

$R^{17}$ ein den Formeln

$-SO_2R^{18}$ oder $-CO\ R^{19}$

entsprechendes Radikal darstellt, in welchen $R^{18}$ eine nicht substituierte oder mit mindestens einem unter den Halogenatomen und den Alkylradikalen ausgewählten Substituenten substituierte Phenylgruppe ist, und

$R^{19}$ ein Wasserstoffatom, $-NH_2$ oder ein, unter den Alkylradikalen, den mit mindestens einer unter den Cyano-, Pyridyl- und $-CO-NH-NH_2$-Gruppen, dem Phenylradikal, dem mit mindestens einem unter -OH, $NH_2$ und den Alkyl- und Alcoxy-Radikalen ausgewählten Substituenten substituiertes Phenylradikal ausgewählten Gruppe substituiertes Alkylradikal, gewähltes Radikal ist.

16. Verfahren gemäß Anspruch 15, dadurch gekennzeichnet, daß $R^{14}$, $R^{15}$ und $R^{16}$ Wasserstoffatome sind, $R^{17}$ das Radikal der Formel $SO_2R^{18}$ darstellt, wobei $R^{18}$ die in Anspruch 15 gegebene Bedeutung hat, oder das Radikal der Formel COH, $CO-CH_2-CH_2-CO-NH-NH_2$ oder

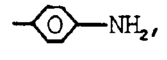

ist und daß man die Reaktion bei Umgebungstemperatur ausführt.

17. Verfahren gemäß Anspruch 16, dadurch gekennzeichnet, daß $R^{17}$ $SO_2R^{18}$ darstellt, wobei $R^{18}$ das Phenylradikal, das p-Methyl-Phenylradikal, das Trichloro-1,3,5-Phenylradikal oder das Trimethyl-1,3,5-Phenylradikal ist.

**18.** Verfahren gemäß einem der Ansprüche 2 bis 17, dadurch gekennzeichnet, daß der zweite Ligand Natrium-Diethyldithiocarbamat ist.

**19.** Verfahren gemäß einem der Ansprüche 2 bis 17, dadurch gekennzeichnet, daß der zweite Ligand zwischen Thioquinolein, Penicillinamin, Natrium-Ethyldithiocarboxylat, Natrium-Isopropylxanthat und Natrium-Diethydithiophosphinat ausgewählt wird.

**20.** Verfahren gemäß einem der Ansprüche 1 bis 19, dadurch gekennzeichnet, daß die Sauerstoffverbindung des Übergangsmetalls Alkalin- oder Ammonium- Metall-Pertechnetat -99m ist.

**21.** Verfahren gemäß einem der Ansprüche 1 bis 19, dadurch gekennzeichnet, daß die Sauerstoffverbindung des Übergangsmetalls Alkalin- oder Ammonium-Metallperrhenat -186 oder -188 ist.

**22.** Ausrüstung für die Herstellung eines Übergansmetallnitridkomplexes durch Einsatz des Verfahrens gemäß einem der Ansprüche 2 bis 21, dadurch gekennzeichnet, daß sie:
- einen ersten Kolben umfaßt, der den ersten Stickstoffliganden und das Reduktionsmittel enthält, und
- einen zweiten Kolben umfaßt, der den zweiten organischen Liganden mit nukleophilen Gruppen, einen monoclonalen Antikörper, ein Antikörperfragment, ein Protein oder ein Peptid enthält.